# EUROPEAN PATENT SPECIFICATION

(11) **EP 2 857 060 B1**
(45) Date of publication and mention of the grant of the patent: **22.02.2017**
(21) Application number: 14173744.5
(22) Date of filing: 24.06.2014
(51) Int. Cl.: A61M 25/09, B21F 7/00

(54) **Shaft and guidewire employing the same**
Schaft und Führungsdraht damit
Arbre et fil-guide utilisant celui-ci

(30) Priority: 02.10.2013 JP 2013207162
(43) Date of publication of application: 08.04.2015
(73) Proprietor: ASAHI INTECC CO., LTD., Nagoya-shi, Aichi 463-0024 (JP)
(72) Inventor: Miyata, Naohiko, Nagoya-shi, Aichi 463-0024 (JP); Furukawa, Muneya, Nagoya-shi, Aichi 463-0024 (JP); Matsuo, Kenichi, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 163 276
- WO-A1-2012/172881
- US-A- 5 299 580
- US-A- 5 313 967
- US-A1- 2004 142 643
- US-A1- 2004 215 109

## Description

### Technical Field

The present invention relates to a shaft employed for a medical apparatus inserted into a body cavity for the purpose of treatment or an exam, and a guidewire employing the shaft.

### Background Art

Conventionally, various medical apparatuses inserted into a tubular organ and body tissue such as a blood vessel, a digestive tract and the ureter have been proposed for the purpose of using for treatment or an exam.

For example, Patent Literature 1 discloses a guidewire including a shaft twisted around a long axis.

### Citation List

### Patent Literature

Patent Document 1: U.S. Patent Application No, 2004/0215109 with the features of the preamble of claim 1.

### Summary of Invention

### Technical Problem

In the case of inserting a conventionally known guidewire along an inverted U-shaped path from the lower extremity vasculature of the right leg into the lower extremity vasculature of the left leg by, for example, the Cross-Over method, when passing through the top of such an extremely winding blood vessel, sliding against the blood vessel wall and the like cause increase in operation resistance of a shaft pushed and pulled. Accordingly, in a conventionally known guidewire, sufficient torque transmission characteristics are not obtained, possibly resulting in reduced operability.

The present invention has been devised in view of such circumstances, and it is an object of the present invention to provide a shaft capable of suppressing the rise in operation resistance when pushed and pulled even inside an extremely winding blood vessel to secure sufficient torque transmission characteristics so that operability is improved, and a guidewire employing the shaft.

### Solution to Problem

In order to solve the above-described problem, a shaft and a guidewire employing the shaft according to one aspect of the present invention have features below.

A shaft according to a first aspect of the present invention is a shaft twisted along a longitudinal direction, having a cross section taking the form of an approximately rectangular shape in a direction vertical to the longitudinal direction, in which the cross section has a pair of projections projected arcuately in a direction vertical to the longitudinal direction, and having the projections formed on a pair of sides facing each other among sides forming the cross section.

A second aspect of the present invention is the shaft according to the first aspect having recesses recessed arcuately on a pair of sides facing each other except those having the projections. in a direction vertical to the longitudinal direction.

A third aspect of the present invention is the shaft according to any one of the first to second aspects having the radius of curvature of the projection smaller than the radius of curvature of a virtual circle having a diameter equivalent to a long axis of the cross section.

A fourth aspect of the present invention is a guidewire including a core shaft and a coiled body covering a distal portion of the core shaft, in which a proximal portion of the core shaft located on a proximal side of the coiled body is the shaft according to any one of the first to third aspects.

A fifth aspect of the present invention is the guidewire according to the fourth aspect having the coiled body composed of a plurality of strands wound helically, each of the strands being made of a core wire and side wires so as to cover a circumference of the core wire.

### Advantageous Effects of Invention

The shaft of an example is twisted along a longitudinal direction and has a cross section with a projection projecting arcuately. Thereby, when the shaft is inserted into a blood vessel, contact areas with a blood vessel wall are reduced due to many grooves generated by twisting, while at least one side among sides forming the cross section is projected arcuately, so that the top of the projected side comes into contact with the blood vessel wall.

That is, comparing to a configuration without a projection projected arcuately (a configuration having a cross-sectional rectangular shape whose four corners are in contact with a blood vessel wall), the shaft of the present invention has reduced contact parts with a blood vessel wall, while having a smaller load applied to the blood vessel wall in contact.

Therefore, when allowing the above shaft to rotate and enter the inside of a blood vessel, contact resistance against a blood vessel wall is reduced. Accordingly, operation resistance of the shaft pushed and pulled is reduced so that torque transmission characteristics are enhanced, resulting in improved operability.

Moreover, even in a case where the above shaft is inserted along an inverted U-shaped path from the lower extremity vasculature of the right leg into the lower extremity vasculature of the left leg by, for example, the Cross-Over method, when passing through the top of a blood vessel, sliding against the blood vessel wall and the like do not cause movement of the shaft to be suppressed, so that a distal portion of the shaft is allowed to be inserted deeply and smoothly into the blood vessel. In addition, it is possible to reduce damage of a blood vessel.

In the shaft of the first aspect, projections are formed on a pair of sides facing each other among sides forming a cross section. Thereby, when inserting the shaft into a blood vessel, top parts of the projections formed on the pair of sides only come into contact with a blood vessel wall. That is, comparing to a configuration without such the projection (a configuration having a cross-sectional rectangular shape whose four corners are in contact with a blood vessel wall), in the shaft of the present invention, contact parts with a blood vessel wall are surely reduced, while a load applied to the blood vessel wall in contact is also further smaller.

Therefore, when allowing the above shaft to rotate and enter the inside of a blood vessel, contact resistance against a blood vessel wall is surely reduced. Accordingly, the shaft of the first aspect brings beneficial results. That is, operation resistance of the shaft pushed and pulled is further reduced so that sufficient torque transmission characteristics are secured, resulting in further enhanced operability.

The shaft of the second aspect has recesses recessed arcuately on a pair of sides facing each other except those having projections. Thereby, comparing to a configuration without sides recessed arcuately. (a shaft having a cross-sectional rectangular shape), moment of inertial of area is lowered. Accordingly, for example, within a blood vessel of a lower extremity region extremely winding in an inverted U-shape, a permanent set tends not to occur even in a case where the shaft excessively bends due to a load applied thereto when coming into contact with a blood vessel wall or the like. Thus, there is no possible trouble in subsequent operation, thereby making it possible to use the shaft continuously.

In the shaft of the third aspect, the radius of curvature of a projection is smaller than the radius of curvature of a virtual circle whose diameter is equivalent to the long axis of a cross section of the shaft. Such the shaft has a cross section in a tapered shape, and has two tops of respective projections in contact with a blood vessel wall.

Therefore, comparing to a configuration without a projection (a configuration having a cross-sectional rectangular shape whose four corners are in contact with a blood vessel wall), in the fourth aspect, contact areas (contact parts) with a blood vessel wall are more surely reduced. Accordingly, when the above shaft rotates and enters the inside of a blood vessel, operation resistance of the shaft pushed and pulled is surely reduced so that sufficient torque transmission characteristics are secured, resulting in further enhanced operability. Furthermore, it is possible to surely reduce damage of a blood vessel.

The guidewire of the fourth aspect includes a core shaft and a coiled body covering a distal portion of the core shaft, in which a proximal portion of the core shaft located on a proximal side of the coiled body is the shaft according to any one of the first to third aspects. Thereby, it is possible to obtain the above-described effect according to the first to fourth aspects. That is, it is possible to insert a distal portion of the guidewire even into an extremely winding blood vessel in an inverted U-shape deeply and smoothly, resulting in enhanced operability. Moreover, operation resistance of the guidewire pushed and pulled is further reduced, while making it possible to reduce damage of a blood vessel effectively.

The guidewire of the fifth aspect includes a coiled body composed of a plurality of strands wound helically, each of the strands being made of a core wire and side wires wound so as to cover a circumference of the core wire. Thereby, comparing to a guidewire including a coiled body composed of, for example, a single wire having an external diameter nearly equal to that of the guidewire of the sixth aspect, flexibility of the coiled body is improved, and it is possible to secure sufficient torque transmission characteristics. Further, breaking strength against twisting is improved, so that safety of the guidewire is enhanced.

### Brief Description of Drawings

Fig. 1 is a general view showing a shaft of a guidewire.
Fig. 2 is a cross-sectional view showing a shaft of an example.
Fig. 3 is a cross-sectional view showing a shaft of a first embodiment of the present invention.
Fig. 4 is a cross-sectional view showing a shaft of a second embodiment of the present invention.
Fig. 5 is a cross-sectional view showing a shaft of a third embodiment of the present invention.
Fig. 6 is a general view showing a first embodiment of a guidewire of the present invention.
Fig. 7 is a general view showing a second embodiment of a guidewire of the present invention.

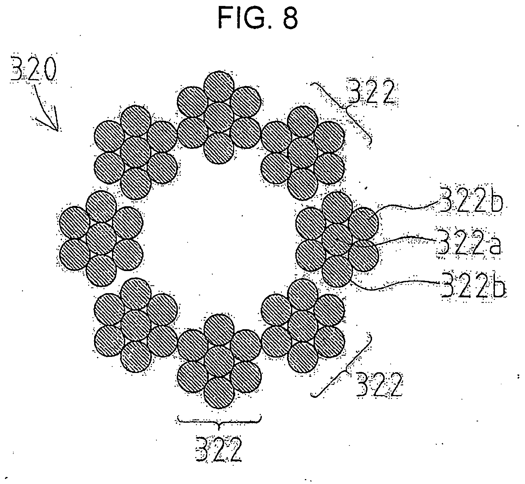

Fig. 8 is a cross-sectional view along A-A of a coiled body in Fig. 7.

### Description of Embodiments

First, description will be given for a shaft of the present invention based on preferred embodiments shown in the drawings.

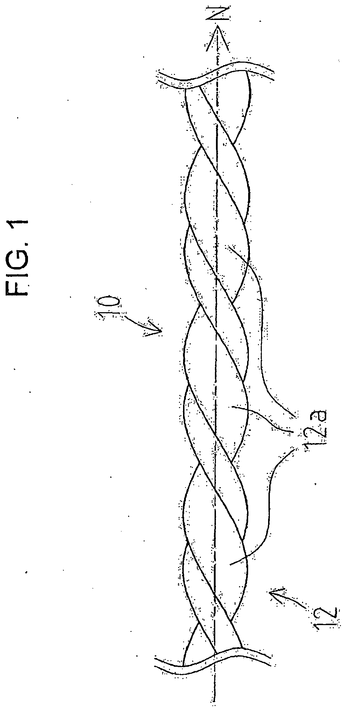

Fig. 1 is a general view showing a example of a shaft of a guide Note that, in this figure, a length direction of the shaft is shortened to be schematically illustrated in whole in order to facilitate the understanding, thus providing an overall dimension different from the actual one.

As shown in Fig. 1, a shaft 10 takes on a rod-like body in an elongated shape. The shaft 10 can be formed using materials such as, for example, stainless steel (SUS304), a super elastic alloy such as an Ni-Ti alloy, and a piano wire, but not especially limited thereto.

The shaft 10 is twisted in a predetermined direction along a longitudinal direction N, and has a helically shaped portion 12a. Additionally, the shaft 10 is provided with a plurality of grooves 12a at regular intervals along the longitudinal direction N. Thereby, for example, when inserting the shaft 10 into a blood vessel, the plurality of grooves 12a leads to reduction of contact areas with a blood vessel wall.

Further, the shaft 10 has the helically shaped portion 12, and thereby, when proximal side of the shaft 10 is rotated, such rotation is easily transmitted to a distal side of the shaft 10. That is, torque transmission characteristics are enhanced, resulting in improved operability.

Moreover, in Fig. 1, a direction of helix of the helically shaped portion 12 is counterclockwise along the longitudinal direction N of the shaft 10. However, the direction of helix of the helically shaped portion 12 is not limited thereto, and may be clockwise along the longitudinal direction N of the shaft 10.

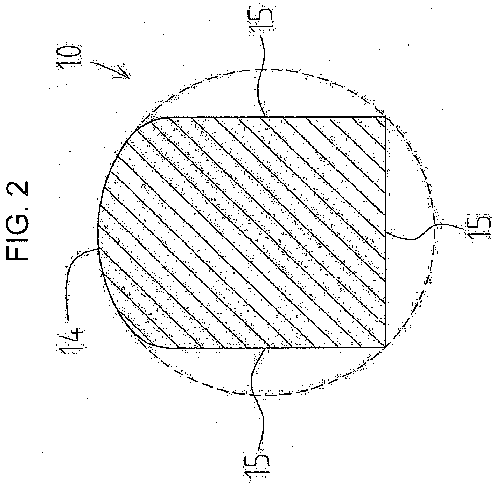

As shown in Fig. 2, the shaft 10 has a cross section in an approximately rectangular shape in a direction vertical to the longitudinal direction N (hereinafter, simply referred to as a cross section). Further, the cross section has a projection 14 projected arcuately. In the present example, the projection 14 is provided only on one side among four sides forming the cross section. Moreover, the other three sides are linear portions 15 formed linearly.

In the example in which the projection 14 is provided on one side among sides forming a cross section, comparing to a configuration without any such the projection 14 (a shaft having a cross-sectional rectangular shape), the shaft 10 has reduced contact parts with a blood vessel wall, while having a smaller load applied to the blood vessel wall in contact.

Therefore, when allowing the shaft 10 to rotate and enter the inside of a blood vessel, contact resistance against a blood vessel wall is reduced. Accordingly, operation resistance of the shaft 10 pushed and pulled is reduced so that torque transmission characteristics are enhanced, resulting in improved operability.

Moreover, even in a case where the above shaft 10 is inserted along an inverted U-shaped path from the lower extremity vasculature of the right leg into the lower extremity vasculature of the left leg by, for example, the Cross-Over method, when passing through the top of a blood vessel, sliding against the blood vessel wall and the like do not cause movement of the shaft 10 to be suppressed, so that a distal portion of the shaft 10 is allowed to be inserted deeply and smoothly into the blood vessel. In addition, it is possible to reduce damage of a blood vessel.

Here, it is possible to fabricate the shaft 10 according to a method described below. First, a columnar metallic body is prepared to be rolled out from a predetermined direction. Thereafter, a rotary device is prepared, capable of applying rotary motion to a circumference of the metallic body having one end fixed, followed by rotation around a long axis of the metallic body from the other end.

Further, a distal end of the metallic body is fixed to one end of the rotary device while a proximal end of the metallic body is fixed to the other end of the rotary device, thereafter applying rotary motion from the other end of the rotary device, so that the metallic body is twisted from the proximal side. Thereby, the shaft 10 is formed, including the helically shaped portion 12 twisted evenly along a longitudinal direction.

The helically shaped portion 12 is twisted by the rotary device, and thereafter has stress applied by the twisting mitigated by means of heat treatment, thereby taking the form of a stable shape. A method of forming the helically shaped portion 12 is not limited to the above-described method, and may be fabricated by the other known method adopted appropriately.

### First Embodiment]

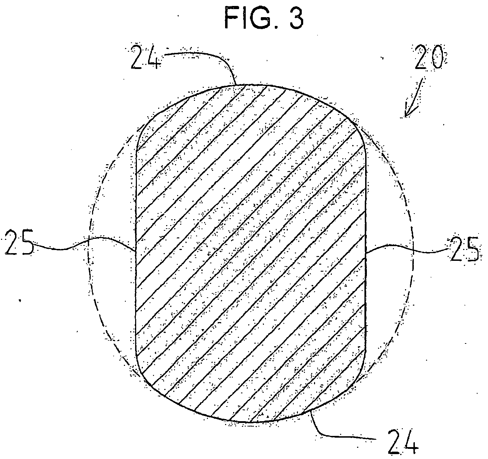

Fig. 3 is a cross-sectional view showing a first embodiment of the shaft of the present invention. Note that, in this figure, a cross section of the shaft is schematically illustrated, thus having a dimensional ratio different from the actual one.

In the shaft 10 of the above-described example, the projection 14 is provided only on one side among sides forming a cross section, and linear portions 15 are provided on the other sides. Whereas, in a shaft 20 of the first embodiment, projections 24 are formed on a pair of sides facing each other. On sides except those having the pair of projections 24, linear portions 25 are provided, respectively.

Thereby, comparing to not only a configuration without a projection (a shaft having a cross-sectional rectangular shape) but also the example in which the projection 14 is provided only on a side among sides forming a cross section, the shaft 20 of the present embodiment has surely reduced contact parts with a blood vessel wall, while having a further smaller load applied to the blood vessel wall in contact.

Therefore, when allowing the above shaft 20 to rotate and enter the inside of a blood vessel, contact resistance against a blood vessel wall is surely reduced. Accordingly, operation resistance of the shaft 20 pushed and pulled is surely reduced so that sufficient torque transmission characteristics are secured, resulting in further improved operability.

### [Second Embodiment]

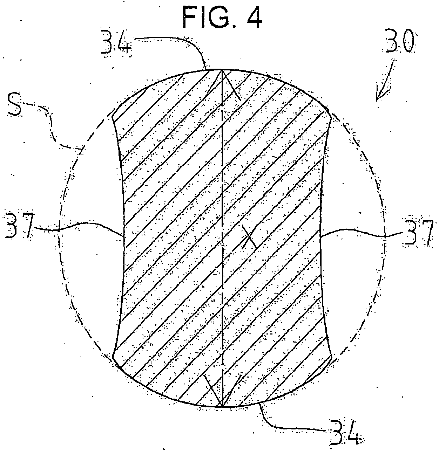

Fig. 4 is a cross-sectional view showing a second embodiment of the shaft of the present invention. Note that, in this figure, a cross section of the shaft is schematically illustrated, thus having a dimensional ratio different from the actual one.

In the shaft 20 of the above-described first embodiment, the projections 24 are formed on a pair of sides facing each other, and the linear portions 25 are respectively provided on sides except those having the pair of the projections 24. Whereas, in a shaft 30 of the second embodiment, on sides except a pair of projections 34 among sides forming a cross section, recesses 37 recessed arcuately are provided, respectively. Note that, in the present embodiment, the radius of curvature of the projection 34 is set so as to be approximately the same as the radius of curvature of a virtual circle S having a diameter equivalent to a long axis X of the cross section.

Thereby, comparing to a configuration without a recess (a shaft having a cross-sectional rectangular shape), moment of inertia of area is lowered. Thus, for example, within a blood vessel of a lower extremity region extremely winding in an inverted U-shape, a permanent set tends not to occur even in a case where the shaft 30 excessively bends due to a load applied thereto when coming into contact with a blood vessel wall, and the like. Accordingly, there is no possible trouble in subsequent operation, thereby making it easy to use the shaft 30 continuously.

### [Third Embodiment]

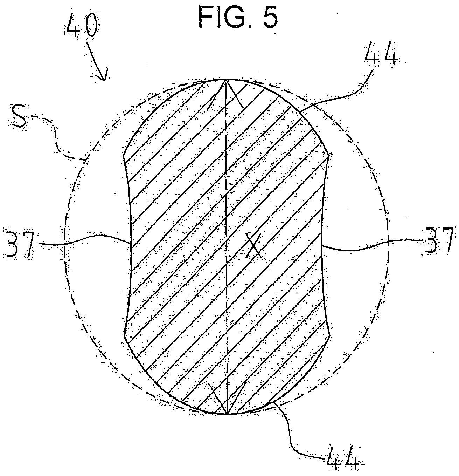

Fig. 5 is a cross-sectional view showing a third embodiment of the shaft of the present invention. Note that, in this figure, a cross section of the shaft is schematically illustrated, thus having a dimensional ratio different from the actual one.

In the above-described second embodiment, the radius of curvature of the pair of the projections 34 is set so as to be approximately the same as the radius of curvature of the virtual circle S having a diameter equivalent to the long axis X of the cross section. Whereas, in a cross section forming a shaft 40 of the present embodiment, the radius of curvature of a pair of projections 44 is smaller than the radius of curvature of the virtual circle S having a diameter equivalent to the long axis X of the cross section.

Such the shaft 40 has a cross section in a tapered shape, and has two tops of respective projections 44 in contact with a blood vessel wall. Therefore, comparing to not only a configuration without a projection (a configuration having a cross-sectional rectangular shape whose four corners are in contact with a blood vessel wall) but also the second embodiment in which a projection is provided, having the radius of curvature approximately the same as the radius of curvature of the virtual circle S, in the shaft 40 of the present embodiment, contact areas (contact parts) with a blood vessel wall are more surely reduced.

Accordingly, when the above shaft 40 rotates and enters the inside of a blood vessel, operation resistance of the shaft 40 pushed and pulled is surely reduced so that sufficient torque transmission characteristics are secured, resulting in further enhanced operability. Furthermore, it is possible to surely reduce damage of a blood vessel.

### [Fourth Embodiment]

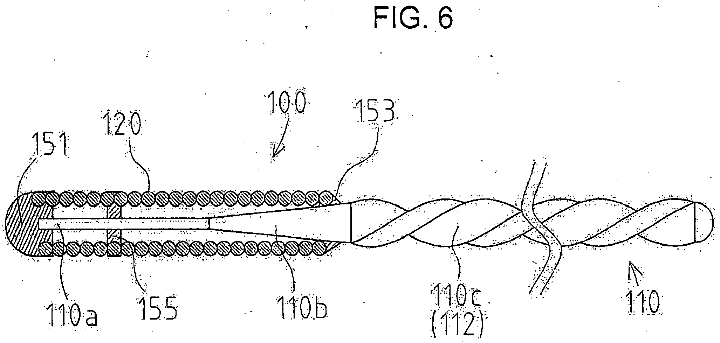

Fig. 6 is a general view showing a first embodiment of a guidewire of the present invention, In Fig. 6, a distal side of the guidewire to be inserted into the body is provided on the left, and a proximal side of the guidewire to be operated by a manipulator such as a doctor is provided on the right. Note that, in this figure, the guidewire is schematically illustrated, thus having a dimensional ratio different from the actual one.

A guidewire 100 shown in Fig. 6 is used for treatment of the lower extremity vasculature by, for example, the Cross-Over method. The guidewire 100 includes a core shaft 110, and a coiled body 120 covering a circumference of a distal portion of the core shaft 110.

First, a description will be given for the core shaft 110. The core shaft 110 includes a small diameter portion 110a, tapered portion 110b and a large diameter portion 110c in order from the distal side to the proximal side. The small diameter portion 110a is a part on the most distal side of the core shaft 110 and the most flexible part of the core shaft 110. The small diameter portion 110a is formed in a tabular shape by press working. The tapered portion 110b has a cross section formed in a tapered round shape whose diameter is gradually reduced toward the distal side. Note that, arrangements and dimensions of the small diameter portion 110a and the tapered portion 110b can be changed appropriately due to reasons to obtain desired rigidity and the like. For example, the small diameter portion 110a may have a columnar shape. Further, the number of the tapered portions 110b and the angle of the tapered portion 110b may also be set appropriately as necessary.

The large diameter portion 110c is located on a proximal side of the coiled body 120, and takes on a shape similar to that of the shaft in the above-described fourth embodiment. That is, the proximal side exposed from the coiled body 120 in the core shaft 110 is twisted along a longitudinal direction, and has a helically shaped portion 112. Moreover, as described in the second embodiment, a cross section of the helically shaped portion 112 has a pair of the projections 34 projected arcuately.

Thereby, comparing to a configuration without any such the projection 34 (a shaft having a cross-sectional rectangular shape), the guidewire 100 of the present embodiment has surely reduced contact areas (contact parts) with a blood vessel wall. Accordingly, when the guidewire 100 rotates and enters the inside of a blood vessel, operation resistance of the guidewire 100 pushed and pulled is surely reduced so that sufficient torque transmission characteristics are secured, resulting in further enhanced operability. Furthermore, it is possible to surely reduce damage of a blood vessel.

Note that, in the guidewire 100 of the present embodiment, the large diameter portion 110c having the same shape as that of the shat of the above-described fourth embodiment is employed, however, the shape of the large diameter portion 110c is not limited thereto. That is, a large diameter portion may be employed having the same shape as that of the shaft of any one of the first to second embodiments. Even in this case, as with the present embodiment, operation resistance of the guidewire 100 pushed and pulled is surely reduced so that sufficient torque transmission characteristics are secured, resulting in further enhanced operability.

The core shaft 110 can be formed using materials such as, for example, stainless steel (SUS304), a super elastic alloy such as an Ni-Ti alloy, and a piano wire, however, not especially limited thereto.

Next, description will be given for the coiled body 120. The coiled body 120 in the present embodiment is a single thread coil composed of wires wound helically.

As shown in Fig. 6, a distal end of the coiled body 120 is fixed a distal end of the core shaft 110 with a distal side joint 151. A proximal end of the coiled body 120 is fixed the core shaft 110 with a proximal side joint 153. Further, an approximately middle part of the coiled body 120 located on a distal side from the proximal side joint 153 and on a proximal side from the distal side joint 151 is fixed the core shaft 110 with a middle joint 155.

Materials forming the distal side joint 151, the proximal side joint 153 and the middle joint 155 are not especially limited, but include, for example, brazing metal such as an Sn-Pb alloy, a Pb-Ag alloy, an Sn-Ag alloy and an Au-Su alloy.

Materials forming the coiled body 120 are not especially limited, but can employ a radiopaque wire or a radiolucent wire. Materials used for a radiopaque wire are not especially limited, but, for example, gold, platinum, tungsten, an alloy containing these elements (for example, a platinum-nickel alloy), or the like. Moreover, materials used for a radiolucent wire are not especially limited, but can include, for example, stainless steel (SUS304, SUS316 and the like), a super elastic alloy such as an Ni-Ti alloy, a piano wire and the like can be used.

### [Fifth Embodiment]

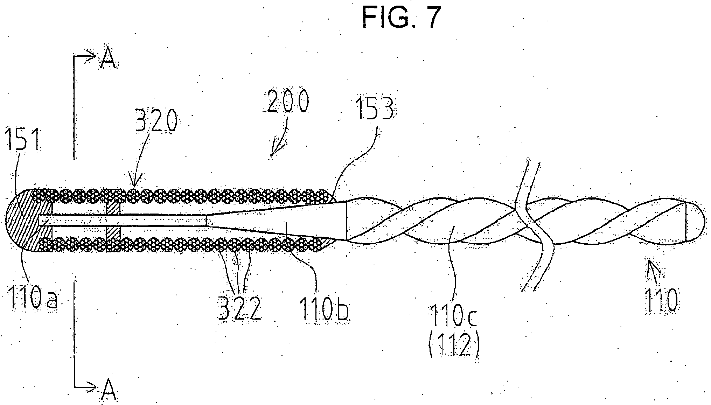

Fig. 7 is a general view showing a second embodiment of a guidewire of the present invention. In Fig. 7, a distal side of the guidewire to be inserted into the body is provided on the left, and a proximal side of the guidewire to be operated by a manipulator such as a doctor is provided on the right. Note that, in this figure, the guidewire is schematically illustrated, thus having a dimensional ratio different from the actual one.

A guidewire 200 of the present embodiment has a configuration of a coiled body different from that of the above-described fourth embodiment. That is, a coiled body 320 employed in the guidewire 200 of the present embodiment is composed of a plurality of strands 322 (eight strands in the present embodiment) wound helically, the strand 322 being made of a core wire (wire) 322a and six side lines (wires) 322b wound so as to cover a circumference of the core wire 322a. Materials forming the core wire 322a and the side line 322b are not especially limited, but include, for example, stainless steel, tungsten, an Ni-Ti alloy and the like.

According to the guidewire 200 of the present embodiment, comparing to a guidewire including a coiled body composed of, for example, a single wire having an external diameter nearly equal to that of the guidewire 200, flexibility of the coiled body is improved, thereby making it possible to secure sufficient torque transmission characteristics. Further, breaking strength against twisting is improved, so that safety of the guidewire 200 is enhanced.

### References Signs List

10,20,30,40...shaft; 14,24,34,44...projection; 37...recess; 100, 200...guidewire; 110...core shaft; 120, 320...coiled body; 322... strand; N...longitudinal direction; X...long axis of a cross section; and S...virtual circle.

## Claims

1. A shaft (20, 30, 40) twisted along a longitudinal direction, having a cross section taking the form of an approximately rectangular shape in a direction vertical to the longitudinal direction, **characterized in that**
the cross section has a pair of projections (24, 34, 44) projected arcuately in a direction vertical to the longitudinal direction, and
the projections (24, 34, 44) are formed on a pair of sides facing each other among sides forming the cross section.

2. The shaft according to claim 1, comprising
recesses (37) recessed arcuately on a pair of sides facing each other except those having the projections in the direction vertical to the longitudinal direction.

3. The shaft according to claim 1 or 2, wherein
the radius of curvature of the projections (44) is smaller than the radius of curvature of a virtual circle having a diameter equivalent to a long axis of the cross section.

4. A guidewire comprising:
a core shaft (110) ; and
a coiled body (120, 320) covering a distal portion of the core shaft, wherein
a proximal portion(110c) of the core shaft located on a proximal side of the coiled body is the shaft according to any one of claims 1 to 3.

5. The guidewire according to claim 4, wherein
the coiled body (320) is composed of a plurality of strands wound helically, each of the strands being made of a core wire and side wires wound so as to cover a circumference of the core wire.

## Patentansprüche

1. Schaft (20, 30, 40), der längs verdrillt ist und in einer Richtung quer zur Längsrichtung einen in etwa rechteckförmigen Querschnitt hat, **dadurch gekennzeichnet, dass**
der Querschnitt zwei Vorsprünge (24, 34, 44) aufweist, die in einer Richtung quer zur Längsrichtung bogenförmig vorspringen, und
die Vorsprünge (24, 34, 44) an zwei einander gegenüberliegenden Seiten der den Querschnitt formenden Seiten gebildet sind.

2. Schaft nach Anspruch 1, mit
Aussparungen (37), die an zwei einander gegenüberliegenden anderen Seiten als den Seiten mit den Vorsprüngen in der Richtung quer zur Längsrichtung bogenförmig ausgespart sind.

3. Schaft nach Anspruch 1 oder 2, wobei
der Krümmungsradius der Vorsprünge (44) kleiner ist als der Krümmungsradius eines virtuellen Kreises mit einem Durchmesser gleich einer Längsachse des Querschnitts.

4. Führungsdraht mit:
einem Kernschaft (110) ; und
einem Wicklungskörper (120, 320), der einen distalen Abschnitt des Kernschafts umgibt, wobei
ein auf einer proximalen Seite des Wicklungskörpers liegender proximaler Abschnitt (110c) des Kernschafts ein Schaft nach einem der Ansprüche 1 bis 3 ist.

5. Führungsdraht nach Anspruch 4, wobei
der Wicklungskörper (320) aus einer Vielzahl von schraubenförmig gewickelten Strängen besteht, die jeweils aus einem Kerndraht und um den Umfang des Kerndrahts herum gewickelten Seitendrähten gebildet sind.

## Revendications

1. Arbre (20, 30, 40) torsadé le long d'une direction longitudinale, ayant une section transversale prenant une forme approximativement rectangulaire dans une direction verticale par rapport à la direction longitudinale, **caractérisé en ce que**
la section transversale a une paire de projections (24, 34, 44) projetées de façon arquée dans une direction verticale par rapport à la direction longitudinale, et
les projections (24, 34, 44) sont formées sur une paire de côtés se faisant face l'un à l'autre entre les côtés formant la section transversale.

2. Arbre selon la revendication 1, comprenant
des renfoncements (37) renfoncés de façon arquée sur une paire de côtés se faisant face l'un à l'autre sauf ceux ayant des projections dans la direction verticale par rapport à la direction longitudinale.

3. Arbre selon la revendication 1 ou 2, dans lequel
le rayon de courbure des projections (44) est plus petit que le rayon de courbure d'un cercle virtuel ayant un diamètre équivalent à un axe long de la section transversale.

4. Fil de guidage comprenant :
un arbre noyau (110) ; et
un corps embobiné (120, 320) couvrant une partie distale de l'arbre noyau, dans lequel
une partie proximale (110c) de l'arbre noyau située sur un côté proximal du corps embobiné est l'arbre selon l'une quelconque des revendications 1 à 3.

5. Fil de guidage selon la revendication 4, dans lequel
le corps embobiné (320) est composé d'une pluralité de torons enroulés de façon hélicoïdale, chacun des torons étant constitué d'un fil d'âme et de fils latéraux toronnés de façon à couvrir une circonférence du fil d'âme.
